# EUROPÄISCHE PATENTSCHRIFT

(11) **EP 1 587 459 B1**
(45) Veröffentlichungstag und Bekanntmachung des Hinweises auf die Patenterteilung: **05.11.2008**
(21) Anmeldenummer: 04701949.2
(22) Anmeldetag: 14.01.2004
(51) Int. Cl.: A61F 2/36, A61F 2/32, A61F 2/30

(54) **HÜFTPROTHESE MIT EINEM IN DEN OBERSCHENKELKNOCHEN EINZUSETZENDEN SCHAFT**
HIP PROSTHESIS COMPRISING A SHAFT TO BE INSERTED INTO THE FEMUR
PROTHESE DE LA HANCHE POURVUE D'UNE TIGE DESTINEE A ETRE ANCREE DANS LE FEMUR

(30) Priorität: 17.01.2003 EP 03001041
(43) Veröffentlichungstag der Anmeldung: 26.10.2005
(73) Patentinhaber: WALDEMAR LINK GmbH & Co. KG, 22339 Hamburg (DE)
(72) Erfinder: KELLER, Arnold, 23863 Kayhude (DE)
(74) Vertreter: Glawe, Delfs, Moll
(86) Internationale Anmeldenummer: PCT/EP2004/000225
(87) Internationale Veröffentlichungsnummer: WO 2004/064689

(56) Entgegenhaltungen:
- EP-A- 0 466 638
- FR-A- 2 602 672
- FR-A- 2 668 059
- FR-A- 2 676 359

## Beschreibung

Für die Verankerung eines Prothesenschaftes im Oberschenkelknochen liegen im metaphysären Bereich andere Bedingungen als im diaphysären Bereich vor. Im metaphysären Bereich, d.h. im wesentlichen im Bereich oberhalb des kleinen Trochanters, ist der weiträumige Knochen von spongiöser Knochensubstanz gefüllt, in welcher für die Aufnahme eines Prothesenschafts ein künstlicher Aufnahmekanal geschaffen werden muß. Da die spongiöse Knochensubstanz weich ist, sind die Kraftübertragungsmöglichkeiten in diesem Bereich begrenzt. Unterhalb des kleinen Trochanters ist der engere Markräum von kräftiger Knochenrinde begrenzt, die für die Kraftübertragung wesentlich bessere Voraussetzungen bietet. Es wurden daher Prothesenschäfte entwickelt, die speziell zur Verankerung und Verkeilung in diesem diaphysären Bereich des Knochens geeignet sind (Schneider: Die Totalprothese der Hüfte, Seite 214 ff.). Ihre verlässliche Verankerung im diaphysären Knochenbereich hat die Folge, daß der metaphysäre Knochenbereich entlastet wird. Wenn Knochensubstanz nicht belastet wird, wird sie allmählich abgebaut. Dies ist unerwünscht.

Bei Prothesen, die primär im metaphysären Bereich des Knochens zu verankern sind, ist es bekannt (EP-B-761183; EP-A-780106; EP-A-1070490; EP-B-159462; EP-B-821923; EP-B-112435; DE-C-4428099), die Verankerung im metaphysären Bereich durch Rippen und Kanten zu verbessern, die von den ventralen bzw. dorsalen Flächen des Prothesengrundkörpers vorspringen. Diese Rippen dürfen eine beträchtliche Ausdehnung in lateromedialer Richtung haben, wenn sie zur Verankerung in Knochenzement oder in einer formgleich ausgefräßten Höhlung bestimmt sind (EP-A-780106; EP-A-1070490; DE-C-4428099). Einen besonders festen postoperativen Sitz der Prothese im Knochen erreicht man jedoch bei zementfreier Implantation dann, wenn Rippen verwendet werden, die bei zementfreier Implantation in die Knochensubstanz einschneiden und sie komprimieren (EP-B-761183). Die vor dem Einsetzen des Prothesenschafts im Knochen vorbereitete Höhlung beschränkt sich dann auf das Volumen des Schaftgrundkörpers. Damit die Rippen beim Eintreiben des Schafts den Knochen nicht sprengen, werden sie schmal und mit schräg abfallenden medialen und lateralen Flanken ausgebildet (EP-B-159462; EP-B-821923; EP-B-761183). Dies beschränkt ihre Fähigkeit zur Kraftübertragung.

Die Erfindung bezieht sich auf diejenigen Prothesengattung, bei der eine Verankerung vornehmlich im diaphysären Bereich des Knochens beabsichtigt ist. Es liegt ihr die Aufgabe zugrunde, dem durch Entlastung verursachten Knochenabbau im methaphysären Bereich entgegenzuwirken.

Die erfindungsgemäße Lösung besteht darin, daß gemäß Anspruch 1 zusätzliche Kraftübertragungsmittel im metaphysären Bereich zur Verfügung gestellt werden, die von Rippen gebildet werden. Diese haben eine besondere Form mit nach medial weisender, steiler Flanke. Die nach vorne bzw. hinten weisende Fläche der Rippe wird dadurch entsprechend breiter als bei den bekannten schmalen Rippen mit schräg abfallenden medialen und lateralen Flanken. Damit trotzdem beim Eintreiben des Schafts in den Knochen keine ihn möglicherweise sprengende Kraft entsteht, ist weiterhin vorgesehen, daß die Rippe von der die mediale Flanke begrenzenden Kante her nach lateral in der Höhe abnimmt. Die herkömmliche, nach lateral gewendete Flanke der Rippen verschwindet dadurch oder wird auf eine verhältnismäßig geringe Höhe, die maximal so groß ist wie die halbe Höhe der medialen Flanke, verringert. Dies ist deshalb tolerabel, weil die nach lateral von der Rippe zu übertragenden Kräfte geringer sind als die nach medial gerichteten.

Das Merkmal, daß die mediale Flanke steil ist, besagt, daß sie einen nahezu rechten Winkel mit der mediolateralen Schaftebene bildet. Sie sollte davon vorzugsweise nicht um mehr als 25°, weiter vorzugsweise mehr als 15°, abweichen.

Die mediale Flanke hat mindestens in einem Teil ihres Verlaufs eine von der Längsrichtung des Schafts abweichende, sich nach oben dem Schenkelhals zuneigende Richtung. Diese Form hat den Vorteil, daß beim Einsetzen der Prothese, wenn die Einschubrichtung mit der Längsrichtung der Prothese übereinstimmt, die mediale Flanke der Rippe wie ein Keil die in Einschubrichtung vor ihr befindliche, spongiöse Knochensubstanz verdichtet, so daß sie zu stärkerer Kraftübertragung befähigt wird. Diese Wirkung wird vornehmlich dann erzeugt, wenn der Winkel, den diese Rippenflanke mit der Längsrichtung des Schafts einschließt, zwischen 5 und 15°, vorzugsweise bei etwa 10° liegt.

Eine entsprechende Kompression auf den anterioren bzw. posterioren Seiten der Rippen kann dadurch erfindungsgemäß erzielt werden, daß die Höhe der Rippen über der jeweiligen Oberfläche des Schaftgrundkörpers von unten nach oben zunimmt. Die Rippe ist somit in doppelter Richtung keilförmig, nämlich erstens zur medialen Seite hin und zweitens zur anterioren bzw. posterioren Seite hin. Nach lateral zu, d.h. auf der der steilen Flanke abgewendeten Seite, kann die Höhe der Rippe allmählich abnehmen.

Die Kompression der spongiösen Knochensubstanz durch die Rippen setzt voraus, daß an der Stelle, an der sich nach der Implantation die Rippen befinden, zuvor Knochensubstanz vorhanden war. Wenn vor dem Einsetzen der Prothese im metaphysären Bereich des Knochens ein Aufnahmekanal für den Prothesenschaft künstlich gebildet wird, soll dieser nur der Querschnittsform des Grundkörpers des Prothesenschafts entsprechen und also noch keine Ausbuchtungen für die spätere Aufnahme der Rippen bilden. Wenn eine Raspel zur Formung dieses Aufnahmekanals verwendet wird, soll sie in ihrer Form also nur dem Schaftgrundkörper entsprechen, ohne Einrichtungen zur Materialabnahme im Rippenbereich aufzuweisen. Alternativ besteht auch die Möglichkeit die Raspel mit Rippen auszurüsten, die den Rippen des Prothesenschaft entsprechen und ohne Materialabnahme zur Kompression der Knochensubstanz eingerichtet sind. Die weiter oben beschriebene Kompression der Knochensubstanz im Kraftübertragungsbereich der Rippen kommt dann schon durch die Raspel zustande oder wird von dieser teilweise vorbereitet.

Die Erfindung wird im folgenden näher unter Bezugnahme auf die Zeichnung erläutert, die ein vorteilhaftes Ausführungsbeispiel veranschaulicht. Es zeigen:
- Fig. 1: eine Ansicht von vorne;
- Fig. 2: eine Ansicht von medial;
- Fig. 3: eine Ansicht von oben; und
- Fig. 4 bis 6: Schnitte durch den Schaft an den entsprechenden Höhen desselben.

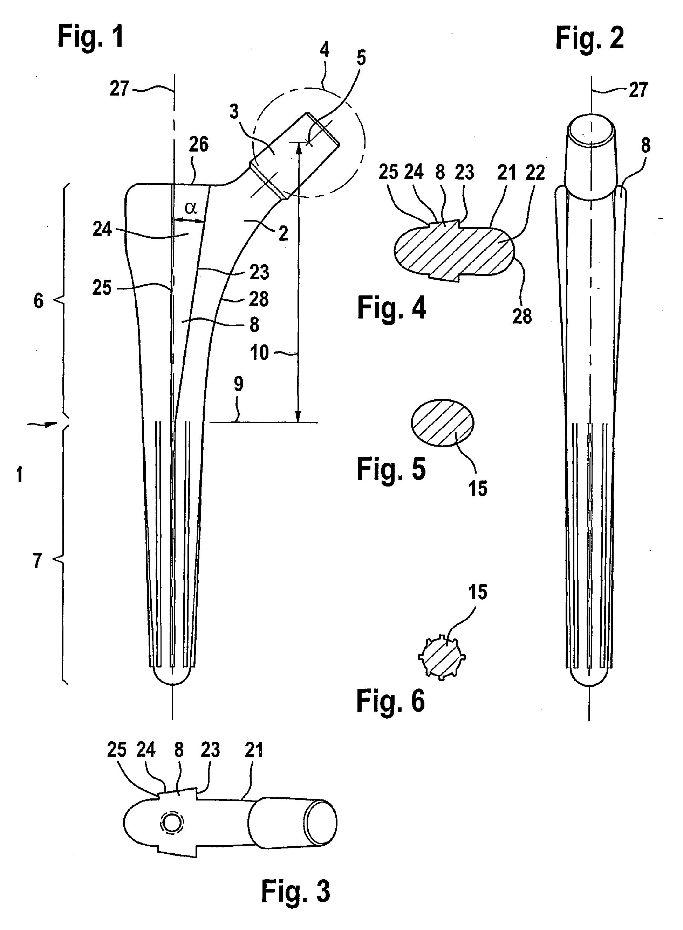

Die Prothese besteht aus einem Schaft 1, einem Hals 2 und einem Konus 3 zum Aufsetzen eines Gelenkkopfs 4, dessen Umfang strichpunktiert angedeutet ist. Der Schaft setzt sich zusammen aus einem proximalen Abschnitt 6 und einem distalen Abschnitt 7. Der proximale Abschnitt ist im Querschnitt langgestreckt in LM-Richtung, wie Fig. 4 und 5 es zeigen. Er ist mit einem Rippenpaar 8 versehen zur Kraftübertragung auf die im epiphysären Bereich des Oberschenkelknochens den Schaft umgebende, spongiöse Knochensubstanz. Der Schaft 1 ist als Geradschaft erkennbar. Das heißt, daß er gerade ausgebildet ist und demzufolge in seiner Längsrichtung und in Längsrichtung der Diaphyse des Oberschenkelknochens in diesen eingetrieben werden muß.

Die Übergangsstelle 9 zwischen dem proximalen und dem distalen Abschnitt des Schafts ist so angeordnet, daß sie im implantierten Zustand etwa beim kleinen Trochanter, vorzugsweise ein wenig unterhalb desselben, zu liegen kommt und der distale Schaftabschnitt 7 demzufolge in einem Bereich des Markkanals liegt, in welchem dieser durch eine starke Knochenrinde begrenzt ist. Die Übergangsstelle braucht an der Prothese nicht besonders markiert zu sein.

Sie ist dadurch bestimmt, daß sie an der Stelle liegt, an der im implantierten Zustand der kleine Trochanter bzw. vorzugsweise dessen Unterkante anzunehmen ist. Sie liegt in der Regel etwa 7 bis 9 cm tiefer als der Mittelpunkt 5 des Gelenkkopfes 4, gemessen gemäß Pfeil 10 in Schaftrichtung.

Der distale Schaftabschnitt ist so ausgebildet, daß er zur Verankerung im diaphysären Bereich des Oberschenkelknochens geeignet ist. Zur Erzielung eines festen Sitzes ist sein Schaftkern schwach konisch und mit Längsrippen besetzt. Dadurch wird Knochensubstanz, die sich im Zwischenraum zwischen der Oberfläche des Schaftkerns 15 und der kortikalen Markraumbegrenzung befindet, komprimiert, wobei sie durch die Rippen festgehalten wird. Der distale Abschnitt kann auch in anderer Weise so ausgebildet sein, daß er zur primären Verankerung des Schafts in der Diaphyse des Knochens geeignet ist.

Die Rippen 8 erheben sich von den anterioren und posterioren Oberflächen 21 des Schaftgrundkörpers 22. Sie weisen eine nach medial gerichtete steile Flanke 23 und einen anterioren bzw. posterioren Oberflächenabschnitt 24 auf, der lateral durch eine Kante 25 begrenzt ist. Die Oberfläche 24 senkt sich mit zunehmender Entfernung von der Flanke 23 zu der Oberfläche 21 hin ab, so daß die Rippe eine etwa dreieckige oder trapezförmige Querschnittsgestalt erhält wie man dies in Fig. 3 und 4 erkennt. Ihre Höhe über der Oberfläche 21 des Grundkörpers 22 ist an der medialen Kante 23 mindestens doppelt so groß wie an der lateralen Kante.

Die Rippen 8 beginnen am Übergang 9 zwischen dem proximalen Abschnitt und dem distalen Abschnitt 7 des Schafts mit der Höhe Null und geringer Breite. Nach oben hin wachsen sie gleichmäßig zu ihrer maximalen Höhe und Breite, die sie am oberen Ende 26 erreichen. Mit der Längsachse 27 des Schafts schließt die Flanke 23 einen Winkel α ein, der im Ausführungsbeispiel bei etwa 8° liegt. Die Höhe der Flanke 23 am oberen Ende 26 des Schafts liegt zwischen 2 und 4 mm, vorzugsweise bei etwa 3 mm. Die Höhe der Flanke 25 liegt zwischen Null und der halben Höhe der Flanke 23. Die Flanke 25 fällt in der Seitenansicht mit der Längsachse 27 zusammen oder verläuft parallel oder in sehr kleinem Winkel dazu.

Die Querschnittsfläche der Rippen vergrößert sich von unten nach oben keilförmig in doppelter Richtung, nämlich zu der Flanke 23 und zu den anterioren bzw. posterioren Flächen 24 hin. Wenn die für die Aufnahme des Prothesenschafts im metaphysären, spongiösen Bereich des Oberschenkelknochens geformte Höhlung im Querschnitt dem Grundkörper 22 des Schafts gleicht, verdrängen die Rippen 8 beim Eintreiben des Schafts das dort befindliche spongiöse Material und verdichten es. Es wird dadurch geeigneter zur Kraftübertragung. Der Abfall der Rippe von der medialen zur lateralen Kante hin hat den weiteren Vorteil, daß der in der Metaphyse des Knochens verfügbare Raum besser für eine voluminöse Schaftausbildung genutzt werden kann.

Das Ausführungsbeispiel zeigt einen linearen Verlauf der Rippen 8. Ihre Keilform kann aber auch einen nicht linearen Verlauf haben.

Wenngleich die Prothese zur primären Verankerung in der Diaphyse bestimmt ist, tragen die Flanken 23 ebenso wie die übrigen nach medial gerichteten Flächen 28 des Prothesen schafts im metaphysären Bereich zur Kraftübertragung bei. Die Metaphyse des Knochens wird dadurch an der Kraftübertragung beteiligt. Die Gefahr ihrer Rückbildung wird dadurch vermindert. Der langzeitig sichere Halt der Prothese im Knochen wird verbessert.

## Patentansprüche

1. Hüftprothese mit einem in den Oberschenkelknochen einzusetzenden Schaft (1), dessen proximaler, im metaphysären Bereich des Oberschenkelknochens einzusetzender Teil (6) auf der Vorder- und Rückseite je eine vorspringende Rippe (8) aufweist, deren mediale Flanke (23) eine von der Längsrichtung (27) des Schafts (1) abweichende, sich nach oben dem Schenkelhals (2) zuneigende Richtung hat, **dadurch gekennzeichnet, daß** die mediale Flanke (23) steil ist und die Höhe der Rippe (8) von der die steile Flanke (23) begrenzenden Kante nach lateral abnimmt.

2. Prothese nach Anspruch 1, **dadurch gekennzeichnet, daß** die Rippe (8) geradlinig unter einem Winkel (Alpha) von 5 bis 15° gegenüber der Längsrichtung (27) des Schafts (1) verläuft.

3. Prothese nach Anspruch 1 oder 2, **dadurch gekennzeichnet, daß** die Höhe der Rippe (8) über der Oberfläche (21) des Schaftgrundkörpers (22) von unten nach oben zunimmt.

4. Prothese nach einem der Ansprüche 1 bis 3, **dadurch gekennzeichnet, daß** die Höhe der lateralen Kante (25) der Rippe (8) nicht größer als die halbe Höhe der medialen Kante ist.

5. Prothese nach einem der Ansprüche 1 bis 4, **dadurch gekennzeichnet, daß** sie diaphysäre Verankerungsmittel aufweist.

## Claims

1. A hip prosthesis comprising a shaft (1) which is to be inserted into the femur and whose proximal part (6) to be inserted in the metaphyseal region of the femur comprises, on the front face and rear face, in each case one projecting fin (8) whose medial flank (23) deviates away from the longitudinal direction (27) of the shaft (1) and at the top is inclined toward the femoral neck (2), **characterized in that** the medial flank (23) is steep, and the height of the fin (8) decreases in the lateral direction from the edge delimiting the steep flank (23).

2. The prosthesis as claimed in claim 1, **characterized in that** the fin (8) extends rectilinearly at an angle (alpha) of 5 to 15° with respect to the longitudinal direction (27) of the shaft (1).

3. The prosthesis as claimed in claim 1 or 2, **characterized in that** the height of the fin (8) above the surface (21) of the main body (22) of the shaft increases from the bottom upward.

4. The prosthesis as claimed in one of claims 1 through 3, **characterized in that** the height of the lateral edge (25) of the fin (8) is not greater than half the height of the medial edge.

5. The prosthesis as claimed in one of claims 1 through 4, **characterized in that** it comprises diaphyseal anchoring means.

## Revendications

1. Prothèse de la hanche pourvue d'une tige (1) destinée à être ancrée dans le fémur, dont la partie proximale (6), destinée à être ancrée dans la région métaphysaire du fémur, présente sur le côté avant et le côté arrière chaque fois une nervure saillante (8) dont le flanc médial (23) a une direction s'écartant de la direction longitudinale (27) de la tige (1) et inclinée vers le haut vers le col du fémur (2), **caractérisée en ce que** le flanc médial (23) présente une pente raide et la hauteur de la nervure (8) diminue en direction latérale à partir de l'arête limitant le flanc à pente raide (23).

2. Prothèse de la hanche selon la revendication 1, **caractérisée en ce que** la nervure (8) est rectiligne sous un angle (alpha) de 5 à 15° par rapport à la direction longitudinale (27) de la tige (1).

3. Prothèse selon la revendication 1 ou 2, **caractérisée en ce que** la hauteur de la nervure (8) au-dessus de la surface (21) du corps de base de la tige (22) augmente de bas en haut.

4. Prothèse selon l'une quelconque des revendications 1 à 3, **caractérisée en ce que** la hauteur de l'arête latérale (25) de la nervure (8) n'est pas plus grande que la moitié de la hauteur de l'arête médiale.

5. Prothèse selon l'une quelconque des revendications 1 à 4, **caractérisée en ce qu'**elle comporte des moyens d'ancrage diaphysaires.
